# EUROPEAN PATENT SPECIFICATION

(11) **EP 4 271 324 B1**
(45) Date of publication and mention of the grant of the patent: **05.08.2026**
(21) Application number: 21916064.5
(22) Date of filing: 28.12.2021
(51) Int. Cl.: A61F 2/16, A61L 31/08, A61L 31/14

(54) **CROSSLINKED POLYMER COATINGS FOR INTRAOCULAR LENS (IOL) CARTRIDGES AND METHOD FOR CREATING THIS COATING ON THE INNER SURFACE OF THE CARTRIDGE**
VERNETZTE POLYMERBESCHICHTUNGEN FÜR INTRAOKULARLINSEN (IOL)-KARTUSCHEN UND VERFAHREN ZUR ERZEUGUNG DIESER BESCHICHTUNG AUF DER INNENFLÄCHE DER KARTUSCHE
REVÊTEMENTS POLYMÈRES RÉTICULÉS POUR CARTOUCHES DE LENTILLES INTRAOCULAIRES (LIO) ET PROCÉDÉ DE CRÉATION DE CE REVÊTEMENT SUR LA SURFACE INTERNE DE LA CARTOUCHE

(30) Priority: 29.12.2020 TR 202022155
(43) Date of publication of application: 08.11.2023
(73) Proprietor: VSY Biyoteknoloji Ve Ilac Sanayi Anonim Sirketi, Tuzla/Istanbul (TR)
(72) Inventor: KAYA, Busra Gizem, Tuzla/Istanbul (TR); OYTUN, Faruk, Tuzla/Istanbul (TR)
(74) Representative: Sevinç, Cenk
(86) International application number: PCT/TR2021/051564
(87) International publication number: WO 2022/146386

(56) References cited:
- WO-A1-2021/133349
- WO-A1-96/24392
- JP-A- 2007 021 010
- US-A1- 2003 134 132
- US-A1- 2005 147 735
- US-A1- 2005 256 528
- HU CAN ET AL: "Preparation and evaluation of a lubricious treated cartridge used for implantation of intraocular lenses", JOURNAL OF BIOMATERIALS SCIENCE. POLYMER EDITION, VSP, UTRECHT, NL, vol. 18, no. 2, 30 November 2006 (2006-11-30), pages 179 - 191, XP009538657, ISSN: 0920-5063, DOI: 10.1163/156856207779116720
- HU CAN, GWON ARLENE, LOWERY MIKE, MAKKER HARISH, GRUBER LAWRENCE: "Preparation and evaluation of a lubricious treated cartridge used for implantation of intraocular lenses", JOURNAL OF BIOMATERIALS SCIENCE, POLYMER EDITION, vol. 18, no. 2, 2007, NL , pages 179 - 191, XP009538657, ISSN: 0920-5063, DOI: 10.1163/156856207779116720

## Description

### Field of the Invention

The present invention relates to crosslinked polymer coatings which provide the lubrication of the inner surface of a cartridge used as an intermediary for the implantation of intraocular lenses (IOL) in order to replace the natural lens with an artificial lens after removal of the natural lens that has lost its transparency in cataract surgery, in order to facilitate the delivery of the intraocular lens.

### Background of the Invention

*Cataract* is the deterioration of vision due to the decrease in the light reaching the retina as a result of the natural crystal lens in the human eye losing its transparency over time due to old age, cortisone drugs, diabetes, excessive use of cigarettes and alcohol, exposure to long-term radiation or sun, impact or several diseases.

The natural crystal lens is located inside the eye, behind the iris, which is the colored part of the eye. Normally, the crystal lens focuses the light on the retina, which enables to send the image to the brain via the optic nerves. However, when the crystal lens becomes blurred due to cataract, it cannot focus properly, the light cannot be collected in a single point and it is scattered. This causes vision problems.

Cataract can be diagnosed with an eye exam. In an eye exam, examination of the eyes is done using a vision test and a slit lamp microscope. By using special eye drops, the pupil is dilated, so that the back of the eye, where the retina and optic nerve are located, can be seen better. If the concerned vision loss cannot be corrected with glasses or contact lenses, surgery may be required to remove the cataract. Cataract surgery involves removing the natural lens that has lost its transparency and replacing it with an artificial lens. The operation is usually performed as an outpatient procedure; it is very safe and effective. The most common type of cataract surgery is known as phacoemulsification (phaco). In this procedure, the doctor makes a small incision in the eye and breaks up the lens using ultrasonic waves. The natural lens is then removed and replaced with an artificial intraocular lens (IOL).

In the state of the art, these intraocular lenses (IOL) were made of polymethyl methacrylate (PMMA) material in the early days due to its biocompatibility. Since PMMA is a hard polymer, a 5-7 mm incision was required for its implantation. Since an incision of this size requires suture, it decreases the comfort of the patient and prolongs the healing process. Furthermore, PMMA must be manually foldable due to its rigid structure and it can only be inserted into the eye using forceps. Since the use of forceps can often damage the optical surface and haptics of the lens, this procedure makes it inevitable to make a wide incision using sutures. In addition, placing the IOL with manual folding requires a technical skill of use.

Recently, cataract surgery can be performed through small incisions with the discovery of innovative surgical instruments, intraocular lenses (IOLs) produced with advanced materials and functions (for example, soft and foldable materials such as silicone, hydrophobic acrylic and hydrophilic acrylic). This incision should be as small as possible to reduce trauma and accelerate recovery.

The development of an injector which can transfer the lens into the eye by opening a small incision has an important meaning for the success of minimal incision cataract surgery. Faster, more controlled and proper placement of the IOL reduces the risk of technical errors due to the implantation procedure, the risk of technical errors associated with IOL or injector damage, and post-operative complications (e.g. infection from microorganism contamination). Implantation performed with smaller incision sizes not only eliminates the need for sutures, but also accelerates the healing process of the patient. For this reason, today, acrylic based foldable, flexible intraocular lenses (IOLs) having both hydrophilic and hydrophobic properties and shape memory are produced. These lenses can be implanted into the eye even in incision sizes of 3 mm or less.

Cartridge injector systems are used for implanting the lens through a small incision into the eye in cataract surgeries. The lens is folded in the cartridge and passed through the small diameter cartridge tunnel, and then unfolded in the lens capsule located in the eye. If necessary, the surgeon can make minor interventions to bring the lens to the suitable position. When the IOL is to be placed into the eye with the injector, it is desired to pass through the tip of the injector undamaged. If an IOL is released from the injector damaged or in the wrong direction, the surgeon must remove the IOL.

IOL cartridges are generally produced from polymers such as polyolefin (e.g. polypropylene) having high hydrophobic properties. When the IOL is pushed inside the cartridge made of these polymers with high friction force, the movement of the lens inside the cartridge is prevented. As the pressure increases, IOL which is folded inside the cartridge has a tendency to expand inside the cartridge with the effect of the friction force, and it becomes possible to come out of the end of the cartridge. In this case, implantation fails and the IOL undergoes physical deformations such as rupture, tear, and scratching.

Recently, different methods have been used to minimize the friction in the cartridge and facilitate the implantation of the intraocular lens (IOL) by coming out of the cartridge tip. One of these methods is adding fatty acid esters such as glycerol monostearate (GMS) to the material of the cartridge as lubricant additive in the production process. The cartridges produced with this method are subjected to high temperatures for the lubricant additive to impregnate into the inner surface of the cartridge. Even though the cartridge added with lubricant provides a quite effective slippery coating, these fatty acid esters (lubricant) rise to the surface of the cartridge over time. This lubricant material, which has risen to the surface during its long shelf life, may adhere to the surface of the intraocular lens (IOL), causing its optical properties to be damaged. Therefore, the shelf life of the cartridges produced by this method should be kept short.

United States patent document no US8323799B2, an application known in the state of the art, discloses that a solution formed by formulating polyvinyl pyrrolidone (PVP) or hyaluronic acid (HA) as a hydrophilic lubricant, commercial urethane dispersion as a matrix polymer and polyfunctional aziridine as a crosslinking agent is used as an IOL cartridge coating material. Within the scope of the said application, it is stated that the cartridges subjected to plasma treatment are coated with the prepared coating solution and then left to dry overnight at 60 °C.

Another method known in the art is to apply a polymer-based lubricant film coating on the inner surface of the cartridge. In patent applications US6238799B1 and US6866936B2 made in accordance with this method, mixtures comprising polyacrylates, polymethacrylates, polyurethanes, polyethylene and polypropylene copolymers, polyvinyl chlorides, epoxides, polyamides, polyesters and alkyd copolymers as matrix polymer; poly(N-vinyl lactams), poly(vinylpyrrolidone), poly(ethylene oxide) polypropylene oxide) polyacrylamides, cellulosic, methyl cellulose, polyacrylic acids, polyvinyl alcohols, and polyvinyl ethers as hydrophilic polymers; and at least one crosslink agent are used as IOL coating material. The coating process is performed by applying this mixture to IOL cartridges. The lubricant coatings disclosed in the said applications are relatively hard and non-flexible. This situation has a risk that the cartridge inside the cartridge may detach from the coated surface due to its hard structure and may damage the lens during the implantation process.

United States patent document no US8821572B2, an application known in the state of the art, discloses that IOL coating material comprises polyurethane and PVP which is a hydrophilic polymer and a cross-linking agent. Here, it is aimed to apply the coating directly to the inner surface of the cartridge as a single layer.

In an article named *"*Preparation and evaluation of a lubricious treated cartridge used for implantation of intraocular lenses", Journal of Biomaterials Science, Polymer Edition, Vol.18, No.2, p.179-191 (2007), Hu et al. disclose that cartridges subjected to plasma treatment are immersed in a coating solution formed by using polyethylene imine (PEI), PVP as IOL coating material and glutaraldehyde as crosslinking agent and cured at °70 C. It is explained that after the lenses are placed in the coated cartridges, viscoelastic gel is injected before implantation to help the lens slide more easily, and it is waited for 4.5 minutes the lens to be activated. This is quite a long time and carries great risks during the surgical operation. Implantation should be performed in a short time after both viscoelastic gel and saline solution, which will ensure that the lens and coating material are activated, are added.

United States patent document no US20170128195A1, an application known in the state of the art, discloses a solution comprising polyurethane and fluorescing sodium salt as IOL cartridge coating material and polyfunctional aziridine which is a crosslinking agent. It is stated that the coated cartridges are exposed to UV light of 254 nm and the indicator properties are observed whether the fluorescent salt showing fluorescent properties is coated homogeneously on the cartridge.

US 2005/256528 Al discloses an insertion device for an intra-ocular lens (IOL) including a channel having an interior surface which contacts the IOL,wherein the channel has a bilaminar coating. The bi laminar coating includes a highly lubricious top coat which is chemically grafted to a base coat, the base coat firmly adhering to the surface of the insertion device. The preferred top coat is an aqueous solution of hyaluronan; the top coat may also include crosslinking agents. This document further discloses a method of enhancing the lubricity of an insertion device for an IOL, the method including applying the bilaminar coating to at least a portion of the insertion device. The interior surface of the device is cleaned by etching followed by surface modification by chemical or plasma treatment, and the formulated base coat is applied by dipping, spraying, brushing, filling or draining or by injection from a pipette and other liquid dispenser. After application of the base coat, the device is heated for a brief period to remove volatiles. The top coat is then applied by methods similar to the ones used for the base coat, and heating resumed to remove volatiles and to bring about the grafting reaction between base coat and top coat.

### Summary of the Invention

The objective of the invention is to develop a coating which will enable the intraocular lens (IOL) to be easily implanted through the cartridge without damaging it, remains stable during its long shelf life, and is flexible and lubricious.

### Detailed Description of the Invention

**"CROSSLINKED POLYMER COATINGS FOR INTRAOCULAR LENS (IOL) CARTRIDGES"** developed in order to fulfil the objective of the present invention is illustrated in the accompanying figures, in which:

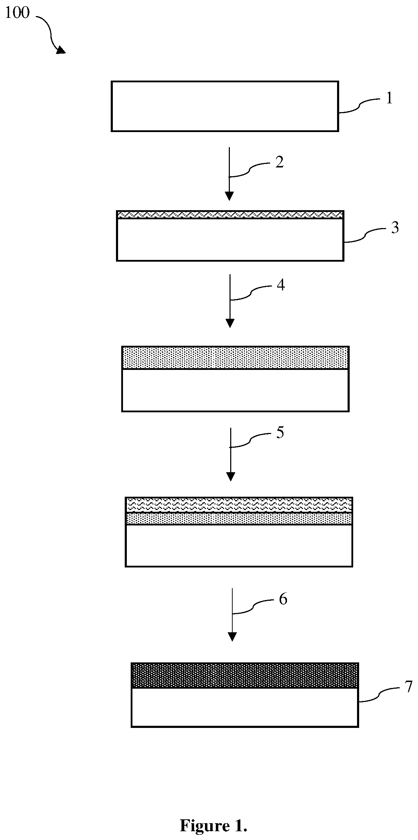

**Figure 1** **-** is a schematic view of the method of applying coating on the surface of intraocular lens (IOL) cartridge within the scope of the invention.
**Figure 2** **-** is a schematic view of the crosslinking reaction within the scope of the invention.

The components shown in the figures are each given reference numbers as follows:
**100.** method of coating the intraocular lens (IOL) cartridge surface (100)
**1.** PP Cartridge
**2.** Plasma Treatment
**3.** PP Cartridge treated with plasma
**4.** Polycation coating
**5.** Polyanion coating
**6.** Curing process
**7.** Coated PP Cartridge

The present invention relates to crosslinked polymer coatings which provide the lubrication of the inner surface, which is particularly in direct contact with the IOL, of a cartridge used as an intermediary for the implantation of intraocular lenses (IOL) in order to replace the natural lens with an artificial lens after removal of the natural lens that has lost its transparency in cataract surgery, in order to facilitate the delivery of the intraocular lens.

The coating method (100) developed within the scope of the invention comprises the steps of
- Applying plasma treatment (2) on the intraocular lens (IOL) cartridge's inner surface, which is particularly in direct contact with the IOL,
- Coating the cartridge surface with the cationic functional polymer as the first layer,
- Then, applying coating on the cationic functional polymer coating on the cartridge surface as a second layer with the solution comprising anionic functional polymer and crosslinker,
- Performing the curing process (6) on the coating surfaces.

Acrijet Fly polypropylene (PP) cartridges (1), produced by VSY Biotechnology, suitable for 2.0 mm micro-incision cataract surgery were subjected to plasma treatment (2) and thereby their surfaces were made hydrophilic. The plasma treatment (2) was carried out for 3 minutes with a gas flow of 0.30 mbar at 100 W power.

The coatings for the intraocular lens (IOL) cartridge developed within the scope of the present invention are crosslinked polymeric coating materials which are used as a coating on the inner surfaces of the intraocular lens cartridges, and which facilitate the implantation of intraocular lenses. The subject matter of the invention enables to develop a coating, which will enable the intraocular lens (IOL) to be easily implanted through the cartridge without damaging it, remains stable during its long shelf life, and is flexible and lubricious.

Within the scope of the invention, two different polymers are used in terms of functional properties. As the first layer, amine functional cationic **polyethyleneimine (PEI)** is used; and as the second layer carboxy and hydroxyl functional anionic polymer **hyaluronic acid (HA)** is used.

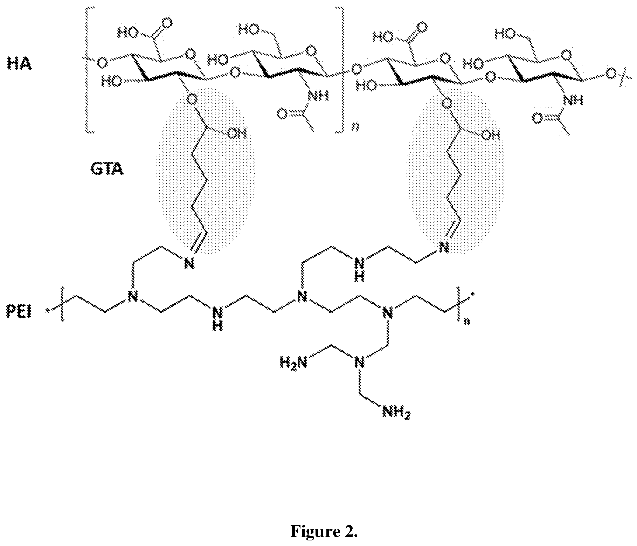

**Glutaraldehyde (GTA)** is used as a crosslinking agent to ensure crosslinking of the polymers among each other. The interaction between the polymers and the crosslinker is shown in Figure 2.

Dip coating method is preferably used as the coating method within the scope of the invention. PP cartridges (1) subjected to the plasma treatment (2) were immersed in PEI solutions for 10 seconds prior to being immersed in the formulations prepared in deionized water, the weight ratios of which are given in Table 1 (in proportion to the total weight of the coating solutions). Cartridges preferably coated with 2.5-10% by weight of PEI were then dried at 80°C for 30 min. The dried cartridges were then immersed in HA + GTA solutions (preferably 0.25-1% HA and 1% GTA by weight) among the formulations mentioned in Table 1 for 10 seconds and left for the crosslinking reaction (curing process (6)) at 80°C for 60 minutes, thereby ensuring the adhesion of the film to the cartridge surface. The GTA ratio was kept constant at 1% by weight in all formulations. After the crosslinking reaction, the PP cartridges (1) were immersed in the ethanol solution so that the impurities remaining or formed in the medium after the reaction were removed; after the removal process the PP cartridges (1) were dried at 60°C for 30 minutes.

**Table 1. Compositions by weight of the solutions used in the coating trials**

| **Trial** | **1^{st} Layer** | **2^{nd} Layer** |
|---|---|---|
| 1 | 2.5% PEI | 0.25% HA |
| | | 1.00% GTA |
| 2 | 2.5% PEI | 0.50% HA |
| | | 1.00% GTA |
| 3 | 2.5% PEI | 1.00% HA |
| | | 1.00% GTA |
| 4 | 5.00% PEI | 0.25% HA |
| | | 1.00% GTA |
| 5 | 5.00% PEI | 0.50% HA |
| | | 1.00 % GTA |
| 6 | 5.00% PEI | 1.00 % HA |
| | | 1.00 % GTA |
| 7 | 10.00% PEI | 0.25 % HA |
| | | 1.00 % GTA |
| 8 | 10.00% PEI | 0.50 % HA |
| | | 1.00 % GTA |
| 9 | 10.00% PEI | 1.00 % HA |
| | | 1.00 % GTA |

The expression "1^{st} layer" indicates the composition of the solution belonging to the first layer in the coating process of the cartridges after the plasma treatment (2). For example, the first layer used in Trial 1 comprises 2.5% PEI relative to the weight ratio of the solution in which it was prepared. After the first coating made with this coating solution, the drying process is carried out and the cartridge becomes coated with the first layer. The expression "2nd layer" indicates the composition of the solution belonging to the second layer of the cartridge coated with the first layer in the coating process. For example, the second layer used in Trial 1 comprises 0.25% HA and 1.00% GTA relative to the weight ratio of the solution in which it was prepared. After the cartridge is coated with the second layer, the curing process (6) is performed and then the impurities are removed in ethanol. As a final step, the cartridges are dried and made ready for use.

Delivery tests of PP cartridges (1) coated with polymer solutions at different ratios, the adhesion properties of the coating to the surface during delivery (whether the coating comes off from the tip of the cartridge with the lens) and the injection force tests were examined.

### EXPERIMENTAL STUDIES

### Delivery Tests

Within the scope of the experimental studies carried out while developing the invention, the delivery tests of hydrophilic acrylic intraocular lenses (IOL) with the cartridges obtained as a result of the curing process (6) were performed. The evaluation of the delivery tests was carried out using the same type of intraocular lenses (Acriva UD 613, VSY Biotechnology) with middle diopter power. The delivery tests were performed with the following process steps:
1. Squeezing viscoelastic gel (Protectalon 1.4 %, VSY Biotechnology) into the coated cartridge
2. Placing the lens in the cartridge
3. Mounting the cartridge into the injector
4. Performing the delivery tests under optic microscope
5. Checking the optical/haptic parts of the lens and checking whether the coating comes off with the lens

The obtained results are summarized in Table 2.

**Table 2. Evaluation of Delivery Tests**

| **Trial** | **Delivery test** | **Observations** | **Coating Transfer** |
|---|---|---|---|
| 1 | ++ | Haptic damage | Not observed |
| 2 | ++ | Haptic damage | Not observed |
| 3 | ++ | Haptic damage | Not observed |
| 4 | +++ | Smooth deliveries, no optic/haptic damage | Not observed |
| 5 | +++ | Smooth deliveries, no optic/haptic damage | Not observed |
| 6 | +++ | Smooth deliveries, no optic/haptic damage | Not observed |
| 7 | +++ | Smooth deliveries, no optic/haptic damage | Not observed |
| 8 | +++ | Smooth deliveries, no optic/haptic damage | Not observed |
| 9 | +++ | Smooth deliveries, no optic/haptic damage | Not observed |

| | | | |
|---|---|---|---|
| +++ Passed ++ Passed with difficulty + Did not pass | | | |

According to the results shown in Table 2, no coating transfer was observed during the delivery from the cartridge tip in any of the delivery trials, and deliveries were successful, except Trials 1, 2 and 3. In Trials 1, 2 and 3, damages occurred in the haptics of the lenses due to the strain during the delivery. In the other trials, the deliveries were performed smoothly, and no deformation was observed in the haptics or optics of the lens. This problem in Trials 1, 2 and 3 can be considered to be due to the fact that the coating is not lubricious enough, that is, the amount of polymer compositions in the coating solution is low. The importance of these polymer compositions, which reduce friction in the cartridge, is clearly seen in the table.

### Injection Force Tests

One of the most important factors during the implantation of IOLs into the eye is the injection force to be applied during the delivery of the lens through the cartridge injector system. Injection force is generally desired to be below 35 N. This is because when high injection force is applied, it becomes difficult to insert the lens into the eye and damages (scratches on the lens, haptic and optic damages) occur in the IOL. In addition, the cartridge injector system may also get damaged (cartridge cracking).

Injection force tests required to be applied during the implantation of Acriva UD 613 IOLs were performed at 200 mm/min using the Lloyd-LS1 test system. In all tests, the middle diopter Acriva UD 613 IOL were used, and Protectalon 1.4% viscoelastic gel was added into cartridges to allow the lens to interact with the coating material and move forward. The results are summarized in Table 3.

**Table 3. Results of injection force applied during delivery tests**

| **Trial** | **Injection Force (N)** |
|---|---|
| 1 | 46.7 |
| 2 | 40.3 |
| 3 | 36.5 |
| 4 | 17.4 |
| 5 | 17.9 |
| 6 | 15.8 |
| 7 | 13.4 |
| 8 | 11.8 |
| 9 | 12.6 |

In the results of the injection force shown in Table 3, the force applied in trials 1, 2 and 3, in which the lens passed through the cartridge with difficulty, exhibited a value above 35 N. In these trials, even though the lens could pass through the cartridge, though with difficulty, it damaged the haptic parts of the lens. This is not suitable for implantation. In other trials (trials 4-9), where the delivery tests were flawless, the injection force results were lower than 35 N and exhibited proper values for flawless implantation. The results of these trials, which showed values well below 35 N, indicate that the coatings performed are suitable for IOL cartridges.

## Claims

1. Crosslinked polymer coatings which provide the lubrication of the inner surface, which is particularly in direct contact with the IOL, of a cartridge used as an intermediary for the implantation of intraocular lenses (IOL) in order to replace the natural lens with an artificial lens after removal of the natural lens that has lost its transparency in cataract surgery, in order to facilitate the delivery of the intraocular lens; wherein the crosslinked polymer coatings contain amine functional cationic polyethyleneimine (PEI) as first layer, carboxylic acid and hydroxyl functional anionic polymer hyaluronic acid (HA) as second layer and glutaraldehyde (GTA) as crosslinking agent.

2. Method of obtaining crosslinked polymer coatings disclosed in claim1, comprising the steps of
- Applying plasma treatment (2) on the intraocular lens (IOL) cartridge's inner surface, which is particularly in direct contact with the IOL,
- Coating the cartridge surface with the cationic amine functional polymer polyethyleneimine (PEI) as the first layer,
- Then, applying coating on the cationic functional polymer coating on the cartridge surface as a second layer with the solution comprising carboxylic acid and hydroxyl functional anionic polymer hyaluronic acid (HA) and glutaraldehyde (GTA) as crosslinker,
- Performing the curing process (6) on the coating surfaces.

3. Method of obtaining crosslinked polymer coatings according to claim 2, wherein the surfaces of the polypropylene (PP) cartridges are subjected to plasma treatment (2) carried out for 3 minutes with a gas flow of 0.30 mbar at 100 W power in order to make them hydrophilic.

4. Method of obtaining crosslinked polymer coatings according to claim 2, wherein 2.5-10% by weight of polyethyleneimine (PEI) polymer is used.

5. Method of obtaining crosslinked polymer coatings according to claim 2, wherein 0.25-10% by weight of hyaluronic acid (HA) polymer is used.

6. Method of obtaining crosslinked polymer coatings according to claim 2, wherein 1-10% by weight of glutaraldehyde (GTA) is used.

7. Method of obtaining crosslinked polymer coatings according to claim 2, wherein PP cartridges (1) subjected to the plasma treatment (2) are immersed in PEI solutions for 10 seconds prior to being immersed in the formulations prepared in deionized water.

8. Method of obtaining crosslinked polymer coatings according to claim 2, wherein the cartridges coated with PEI are then dried at 80°C for 30 min.

9. Method of obtaining crosslinked polymer coatings according to claim 2, wherein cartridges coated with PEI are then immersed in solutions comprising HA and GTA for 10 seconds.

10. Method of obtaining crosslinked polymer coatings according to claim 2, wherein the cartridges immersed in solutions comprising HA and GTA are left for the curing process (6) which is a crosslinking reaction at 80°C for 60 minutes.

11. Method of obtaining crosslinked polymer coatings according to claim 2, wherein, after the crosslinking reaction, the PP cartridges (1) are immersed in ethanol solution so that the impurities remaining or formed in the medium after the reaction are removed, and then the PP cartridges (1) are dried at 60°C for 30 minutes.

## Patentansprüche

1. Vernetzte Polymerbeschichtungen, die für die Schmierung der inneren Oberfläche, die insbesondere in direktem Kontakt mit der IOL steht, einer Patrone sorgen, die als Zwischenprodukt für die Implantation von Intraokularlinsen (IOL) verwendet wird, um die natürliche Linse durch eine künstliche Linse zu ersetzen, nachdem die natürliche Linse, die ihre Transparenz bei einer Kataraktoperation verloren hat, entfernt wurde, um die Einbringung der Intraokularlinse zu erleichtern; wobei die vernetzten Polymerbeschichtungen enthalten
aminfunktionelles kationisches Polyethylenimin
(PEI) als erste Schicht, carboxylsäure- und hydroxylfunktionelles anionisches Polymer Hyaluronsäure (HA) als zweite Schicht und Glutaraldehyd (GTA) als Vernetzungsmittel enthalten.

2. Verfahren zur Herstellung von vernetzten Polymerbeschichtungen nach Anspruch 1, umfassend die Schritte
- Aufbringen einer Plasmabehandlung (2) auf die innere Oberfläche der Intraokularlinsen (IOL)-Patrone, die insbesondere in direktem Kontakt mit der IOL steht,
- Beschichtung der Patronenoberfläche mit dem kationischen aminfunktionellen Polymer Polyethylenimin (PEI) als erste Schicht,
- Beschichtung der Kartuschenoberfläche mit dem kationischen Funktionspolymer als zweite Schicht mit der Lösung aus Carbonsäure und hydroxylfunktionellem anionischem Polymer Hyaluronsäure (HA) und Glutaraldehyd (GTA) als Vernetzer,
- Durchführen des Härtungsprozesses (6) auf den Beschichtungsoberflächen.

3. Verfahren zur Herstellung von vernetzten Polymerbeschichtungen nach Anspruch 2, bei dem die Oberflächen der Polypropylen (PP)-Kartuschen einer Plasmabehandlung (2) unterzogen werden, die 3 Minuten lang mit einem Gasfluss von 0,30 mbar bei 100 W Leistung durchgeführt wird, um sie hydrophil zu machen.

4. Verfahren zur Herstellung von vernetzten Polymerbeschichtungen nach Anspruch 2, wobei 2,5-10 Gew.-% Polyethylenimin (PEI)-Polymer verwendet werden.

5. Verfahren zur Herstellung von vernetzten Polymerbeschichtungen nach Anspruch 2, wobei 0,25-10 Gew.-% eines Hyaluronsäure (HA)-Polymers verwendet werden.

6. Verfahren zur Herstellung von vernetzten Polymerbeschichtungen nach Anspruch 2, wobei 1-10 Gew.-% Glutaraldehyd (GTA) verwendet werden.

7. Verfahren zur Herstellung von vernetzten Polymerbeschichtungen nach Anspruch 2, wobei PP-Kartuschen (1), die der Plasmabehandlung (2) unterzogen werden, 10 Sekunden lang in PEI-Lösungen eingetaucht werden, bevor sie in die in entionisiertem Wasser hergestellten Formulierungen eingetaucht werden.

8. Verfahren zur Herstellung von vernetzten Polymerbeschichtungen nach Anspruch 2, wobei die mit PEI beschichteten Kartuschen anschließend 30 Minuten lang bei 80 °C getrocknet werden.

9. Verfahren zur Herstellung von vernetzten Polymerbeschichtungen nach Anspruch 2, wobei mit PEI beschichtete Kartuschen anschließend 10 Sekunden lang in HA und GTA enthaltende Lösungen getaucht werden.

10. Verfahren zur Herstellung von vernetzten Polymerbeschichtungen nach Anspruch 2, bei dem man die in HA und GTA enthaltende Lösungen getauchten Kartuschen dem Härtungsprozess (6) überlässt, der eine Vernetzungsreaktion bei 80°C für 60 Minuten ist.

11. Verfahren zur Herstellung von vernetzten Polymerbeschichtungen nach Anspruch 2, wobei die PP-Kartuschen (1) nach der Vernetzungsreaktion in eine Ethanollösung getaucht werden, so dass die nach der Reaktion im Medium verbliebenen oder gebildeten Verunreinigungen entfernt werden, und dann die PP-Kartuschen (1) 30 Minuten lang bei 60°C getrocknet werden.

## Revendications

1. Revêtements polymères réticulés qui assurent la lubrification de la surface interne, qui est particulièrement en contact direct avec la LIO, d'une cartouche utilisée comme intermédiaire pour l'implantation de lentilles intraoculaires (LIO) afin de remplacer le cristallin naturel par une lentille artificielle après ablation du cristallin naturel qui a perdu sa transparence lors d'une chirurgie de la cataracte, afin de faciliter la mise en place de la lentille intraoculaire ; dans lesquels les revêtements polymères réticulés contiennent
du polyéthylèneimine (PEl) cationique à fonction amine
comme première couche, de l'acide carboxylique et de l'acide hyaluronique (HA) polymère anionique à fonction hydroxyle comme deuxième couche et du glutaraldéhyde (GTA) comme agent de réticulation.

2. Procédé d'obtention de revêtements polymères réticulés décrit dans la revendication 1, comprenant les étapes suivantes :
- Application d'un traitement au plasma (2) sur la surface interne de la cartouche de lentille intraoculaire (LIO), qui est particulièrement en contact direct avec la LIO,
- Revêtement de la surface de la cartouche avec le polymère polyéthylèneimine (PEl) à fonction amine cationique comme première couche,
- Puis, application d' un revêtement sur le revêtement polymère à fonction cationique à la surface de la cartouche en tant que deuxième couche avec la solution comprenant de l'acide carboxylique et un polymère anionique à fonction hydroxyle, l'acide hyaluronique (HA) et le glutaraldéhyde (GTA) en tant qu'agent de réticulation.
- Réalisation du processus de durcissement (6) sur les surfaces revêtues.

3. Procédé d'obtention de revêtements polymères réticulés selon la revendication 2, dans lequel les surfaces des cartouches en polypropylène (PP) sont soumises à un traitement au plasma (2) effectué pendant 3 minutes avec un débit de gaz de 0.30 mbar à une puissance de 100 W afin de les rendre hydrophiles.

4. Procédé d'obtention de revêtements polymères réticulés selon la revendication 2, dans lequel on utilise 2.5 à 10 % en poids de polymère polyéthylèneimine (PEI).

5. Procédé d'obtention de revêtements polymères réticulés selon la revendication 2, dans lequel on utilise 0.25 à 10 % en poids de polymère d'acide hyaluronique (HA).

6. Procédé d'obtention de revêtements polymères réticulés selon la revendication 2, dans lequel on utilise 1 à 10 % en poids de glutaraldéhyde (GTA).

7. Procédé d'obtention de revêtements polymères réticulés selon la revendication 2, dans lequel les cartouches en PP (1) soumises au traitement au plasma (2) sont immergées dans des solutions de PEI pendant 10 secondes avant d'être immergées dans les formulations préparées dans de l'eau déionisée.

8. Procédé d'obtention de revêtements polymères réticulés selon la revendication 2, dans lequel les cartouches revêtues de PEI sont ensuite séchées à 80 °C pendant 30 minutes.

9. Procédé d'obtention de revêtements polymères réticulés selon la revendication 2, dans lequel les cartouches revêtues de PEI sont ensuite immergées dans des solutions comprenant de l'HA et du GTA pendant 10 secondes.

10. Procédé d'obtention de revêtements polymères réticulés selon la revendication 2, dans lequel les cartouches immergées dans des solutions comprenant de l'HA et du GTA sont laissées pour le processus de durcissement (6) qui est une réaction de réticulation à 80 °C pendant 60 minutes.

11. Procédé d'obtention de revêtements polymères réticulés selon la revendication 2, dans lequel, après la réaction de réticulation, les cartouches en PP (1) sont immergées dans une solution d'éthanol afin d'éliminer les impuretés restantes ou formées dans le milieu après la réaction, puis les cartouches en PP (1) sont séchées à 60 °C pendant 30 minutes.
